# EUROPEAN PATENT SPECIFICATION

(11) **EP 1 338 275 B1**
(45) Date of publication and mention of the grant of the patent: **29.02.2012**
(21) Application number: 01998334.5
(22) Date of filing: 28.11.2001
(51) Int. Cl.: A61K 9/52, A61K 9/22, A61J 3/07

(54) **PHARMACEUTICAL COMPOSITION, THE USE THEREOF AND METHOD FOR PRODUCING SAID COMPOSITION**
PHARMAZEUTISCHE ZUSAMMENSETZUNG, IHRE VERWENDUNG UND VERFAHREN ZUR HERSTELLUNG DIESER ZUSAMMENSETZUNG
COMPOSITION PHARMACEUTIQUE, SON APPLICATION ET PROCEDE DE FABRICATION

(30) Priority: 28.11.2000 RU 2000129647; 20.08.2001 EA 200100794
(43) Date of publication of application: 27.08.2003
(73) Proprietor: Nartsissov, Yaroslav, Moscow, 115304 (RU)
(72) Inventor: Komissarova, Irina Alexeevna, (RU)
(74) Representative: Reynolds, Julian David
(86) International application number: PCT/RU2001/000513
(87) International publication number: WO 2002/043710

(56) References cited:
- RU-C1- 2 025 121
- SU-A3- 1 535 369
- US-A- 4 016 254
- US-A- 4 539 315
- US-A- 4 867 987
- US-A- 5 560 928
- US-A- 5 767 107
- IVANOVOI L.A.: 'Tekhnologiya lekarstvennykh sredstv' MEDITSINA vol. 2, 1991, MOSCOW, pages 169 - 170, 182, XP002976076

## Description

The present invention relates to the field of chemical pharmaceutical industry, namely, to pharmaceutical formulations for manufacturing of prolonged release tablets, in particular, tablets for sublingual administration, and to methods of preparation of such pharmaceutical formulations.

There is known a prolonged release drug dosage form comprising capsule filled with microcapsules containing active substance (pharmaceutical drug). Microcapsules are formed by active principle deposited on insoluble core and coated with polymeric layer. This dosage form is intended for oral administration and has estimated disintegration time of 15 hours. Structure of the drug dosage form and its disintegration time prevent from its utilization in the drug dosage forms for sublingual application. Method of microcapsules manufacture provides for no features allowing regulation of disintegration time of the drug dosage form. Regulation of disintegration time is only possible by the way of changing microcapsule parameters and its coating.

There are known formulations for tablets manufacturing (USA patent 3,873,713, cl.424/184, 1975; USA patent 4,016,254, cl.424/497, 1977) comprising microcapsules made of the drug crystals coated with polymer material layer. Tablets are pressed from microcapsules compounded with auxiliary substances (excipients). Methods of preparation of the formulation comprise deposition of coatings of film-forming substances on the drug crystals, and addition of lubricants followed with tablets compressing. These solutions are not concerned with a problem of disintegration time regulation of the dosage form. Disintegration time is determined by structure of the dosage form obtained and addition of standard auxiliary substances.

The present invention is aimed at creation of pharmaceutical formulation for manufacturing of the prolonged release tablets having disintegration time up to 30 minutes and method for preparation of such formulation, which allows to regulate tablet disintegration time in the said range, in particular, on their sublingual administration, using pharmaceutical drug and water.

To solve this problem, pharmaceutical formulation is proposed comprising microcapsules, each being non-agglomerated particle of water soluble pharmaceutical drug having polymer coating of film-forming substance, dispersant (pharmaceutical drug crystals), and water.

In accordance with the present invention, said pharmaceutical formulation is made up at following components ratio, % wt.:

| | |
|---|---|
| microcapsules | 96.0 - 99.8 |
| lubricant | 0.1 - 1 |
| water | 0.3 - 1 |

at dispersant content of 0.1 to 10 % wt. of the mixture obtained, the pharmaceutical drug and film-forming substance content in microcapsule amounting to 96.0 to 99.2 % wt. and 0.8 to 4 % wt., correspondingly.

In accordance with the present invention, non-agglomerated particles of water soluble pharmaceutical drug used for film coating have dimensions of 200 to 700 micron.

Utilization of the larger particles allows to slow down dissolution process, and also to diminish concentration of auxiliary substances necessary for compressing the tablets. By changing the particles size, disintegration and tablet dissolution rate may be varied. In accordance with the invention, as a rule, the amount of particles having size below 200 micron shouldn't exceed 10% of the bulk substance.

When preparing the formulation, microcapsules are made beforehand by applying a coating layer of natural or synthetic polymers on non-agglomerated particles of water soluble pharmaceutical drug. In the process of microcapsules preparation water content in the coating is brought to concentration below 0.5 % wt. Reduction in moisture content of microcapsules is also possible during their intermediate storage.

To prepare pharmaceutical formulation of this invention, microcapsules having moisture content below 0.5 % wt. are moistened to the water content of 0.1 to 3 % wt. The moistening is carried out to swelling of polymeric coating of the microcapsules. After that, lubricant is introduced, and then dispersant as fine fraction of the pharmaceutical drug.

Drying and subsequent moistening of microcapsules in the course of tablet mixture preparation impart more regular form to the microcapsules coating and exert positive effect on structure homogeneity of the dosage forms manufactured, providing for possibility of stabilization of the tablets disintegration indices.

Water content in the range of 0.1 to 3 % wt. provides for the swelling of polymeric coating and results in the structure of the polymeric coating, which was of flaky texture after deposition in fluidized bed apparatus, becoming a continuous one. Such structure of polymeric coating is preserved in the tablet obtained, ensuring increase in the tablet disintegration time due to decrease in the coating permeability.

Introduction of dispersant into the formulation imparts porosity to the tablets and facilitates a decrease in their disintegration time. At the same time, change of the dispersant concentration and utilization of fine fraction of the pharmaceutical drug as dispersant allow to regulate disintegration time of the dosage form and to maintain high content of the pharmaceutical compound in the preparation without addition of traditional adjuvants.

In accordance with this invention, pharmaceutical drug serves as dispersant. In such a way, both the substance being the active principle of the tablet and the dispersant have identical physical parameters. Owing to this reason (in particular, to equal solubilities of dispersant and pharmaceutical drug in the formulation of microcapsule) gradual dissolution of the tablet is achieved with release of the pharmaceutical substance rather than fast disintegration into its constituent particles, without increase in total dissolution surface.

To obtain coating, cellulose ethers may be used, which are soluble in water or mixture of water with organic solvent.

Coating layer is deposited on the non-agglomerated particles of pharmaceutical drug in fluidized bed apparatus.

As lubricant, stearic acid or its pharmaceutically acceptable salts, as well as mixtures of stearic acid and its salts.

As pharmaceutical drug, aminoacetic acid or xylitol may be used, or other crystalline water-soluble substances.

To obtain pharmaceutical formulation of this invention for manufacturing of the dosage form of preparation, in particular, comprising as pharmaceutical drug aminoacetic acid (crystalline water-soluble substance), at first microcapsules are prepared, each being a non-agglomerated particle of aminoacetic acid coated with a shell of the film-forming substance, in which capacity, for example, methylcellulose is used, as follows:
preparation of crude material for microcapsules is accomplished using 1.24 kg of methylcellulose per 100 kg of aminoacetic acid;
1.2% aqueous solution of methylcellulose is prepared in the reactor equipped with heating jacket and mixer or in reservoir with hand stirring, the solution obtained is left to stand until full swelling of methylcellulose, and then cooled and filtered to remove clots;
preparation of pharmaceutical drug is carried out by sifting of crude material to separate particles of specified size;
aminoacetic acid is brought into fluidized state, and then methylcellulose solution is fed into fluidized bed apparatus at 35-42 °C with air-spraying nozzle to secure deposition of methylcellulose coating on non-agglomerated particles of aminoacetic acid;
after completing the feeding of methylcellulose solution, the bulk is dried to residual moisture of 0.5 % wt.; and
product is discharged and separated from agglomerates and lumps. At that, specified fraction may be isolated.

The finished product obtained as described above is loaded into containers for temporary storage before further processing. Methylcellulose content in the finished product equals to 1±0.1%, and moisture - up to 0.5% of the total weight.

After that, mixture for tablets pressing (tableting mixture) is compounded on basis of the microcapsules prepared. The compounding of this mixture is accomplished by 10 kg batches. To this purpose, strained microcapsules of 200 to 700 micron size are moistened in mixer with water and then placed in closed container for a time period sufficient for swelling of the polymeric coating and formation of semi-product with residual moisture of 0.2 to 0.8 % wt.

After moistening, the bulk material is prepared for tableting. To this purpose, a part of the moistened material (about 1 kg) is picked up and mixed in separate container with lubricant, in which capacity magnesium stearate may be used, introduced in small portions with thorough mixing. At that, total weight of the lubricant amounts usually to 0.097 kg. After that, mixture comprising microcapsules and lubricant is blended with remainder (about 9 kg) of moistened microcapsules.

To regulate tablet disintegration time, dispersant is introduced to tableting mixture in the amount of 0.1-10%, that is, 10 to 100 g of dispersant may be used per 1 kg of bulk material. As dispersant, fine fraction of pharmaceutical drug is used, which is an active principle.

After that, bulk material prepared in such a way is sifted. Sifting may be performed also after introduction of lubricant, prior to dispersant. Both components may be also introduced after sifting.

The tableting mixture prepared is pressed with automated tablet-compressing machine equipped with punch of 6 mm diameter providing for manufacturing of tablet having weight of 0.102±7.5%

In the process of compression mean tablet weight, tablets dimensions, their appearance, and disintegration times are checked at least once every hour. On deviation of the press from these parameters, loading and weight adjustment is performed.

Tablet manufactured as described above, comprise aminoacetic acid - 0.1 g, water-soluble methylcellulose - 0.001 g, and magnesium stearate (magnesium salt of stearic acid) - 0.001 g.

Mean weight of the tablet obtained is 0.102 g ± 7.5%, with tablet being of plano-cylindrical form with 2.6±0.3 mm height and 6±0.2 mm diameter. Tablets prepared by the method of this invention satisfy established specifications as to appearance. Typical of the tablet obtained is white coloring with mottled elements.

Disintegration time of the aminoacetic acid tablets obtained amounts to up to 30 min.

The process flow described is common for different water-soluble crystalline pharmaceutical substances, and in certain cases individual parameters may vary, such as fluidization temperature, concentration of the moistening liquid, and treatment time for crystals coating. Utilization of pharmaceutical drug particles of 200 to 700 micron size ensures sublingual application of the tablets manufactured as described above.

Feasibility of realization of this invention is confirmed with following examples.

### Example 1

100 kg of aminoacetic acid are placed into fluidized bed apparatus for deposition of coating. The substance is moistened with 1.2 % solution of methylcellulose (M-100 grade) for 4-5 hours at 42 °C. After completion of the process, product is dried to residual moisture of 0.1 % wt., dusted with magnesium stearate and mixed with dispersant, comprising fine fraction of the active principle, in the quantity of 5 % wt.

Composition of the obtained semi-product - mixture for tablets pressing (tableting mixture) is presented in Table 1. Corresponding physicochemical parameters for the tablets (6 mm, 0.102 g) are listed in Table 2.

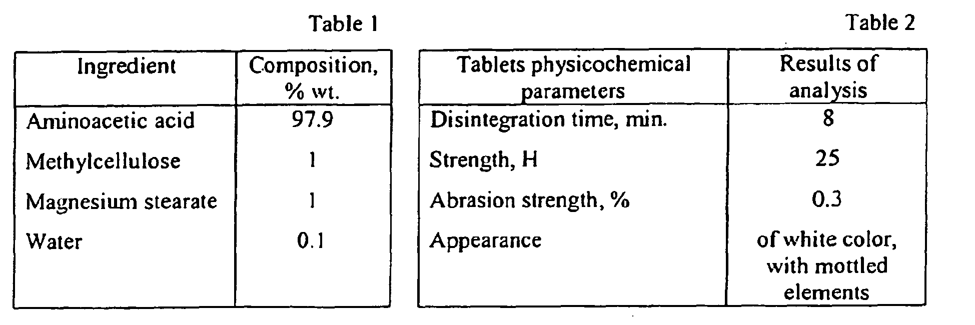

### Example 2

Microcapsules prepared analogous to Example 1, are moistened to water content of 0.7 % wt., dusted with magnesium stearate, after which fine fraction of the active principle is introduced in the amount of 5 % wt. Composition of the formulation obtained is listed in Table 3. Physicochemical parameters of the corresponding tablets (6 mm, 0.102 g) are presented in Table 4.

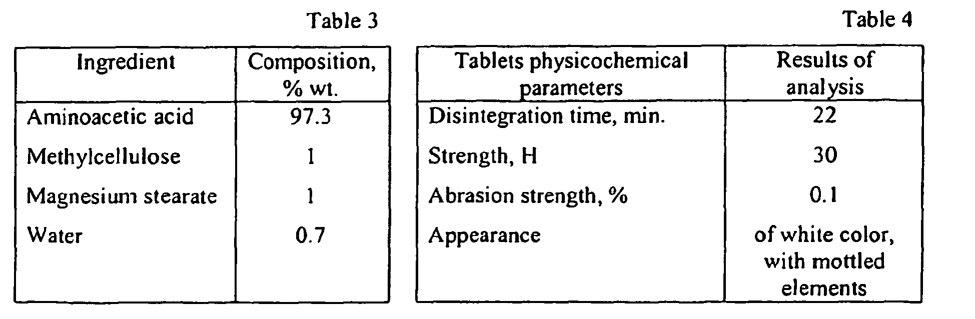

### Example 3

100 kg of xylitol are placed into fluidized bed apparatus. To deposit film coating, the substance is moistened with 3.0 % solution of methylcellulose (M-100 grade) in a manner analogous to Example 1. After completion of the process, product is dried to moisture content not more than 0.1 % wt. and dusted with magnesium stearate. Then dispersant is introduces analogous to Example 1. Composition of the formulation obtained is presented in Table 5, and physicochemical parameters of corresponding tablets - in Table 6.

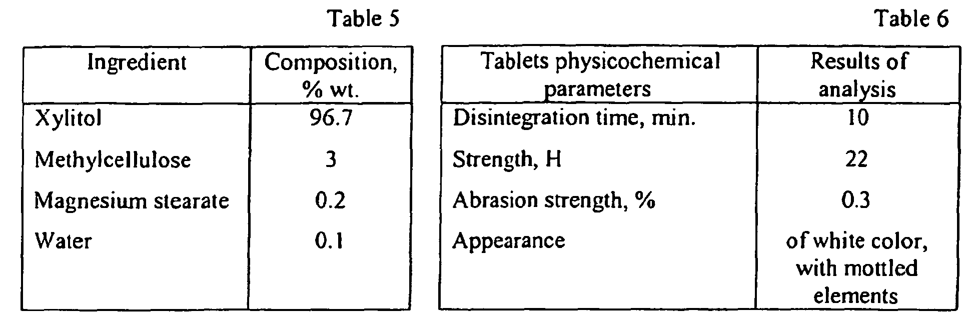

### Example 4

Semi-product obtained analogous to Example 3 is moistened to moisture content of 0.3 % wt. and dusted with magnesium stearate. Composition of the formulation obtained is presented in Table 7, and corresponding physicochemical properties - in Table 8.

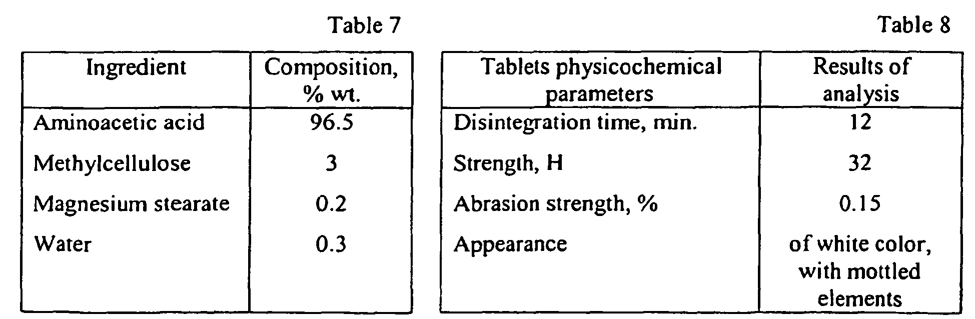

### Example 5

100 kg of aminoacetic acid is placed into fluidized bed apparatus for deposition of film coating. The substance is moistened with 3% solution of methylcellulose (M16 grade) in a manner analogous to the one described in Example 1.

After completion of the process, product is dried to moisture content of 3 % wt., dusted with 1 % wt. of magnesium stearate, and mixed with dispersant 10 % wt. Composition of the formulation obtained (tableting mixture) is presented in Table 9, and physicochemical properties of the tablets (6 mm, 0.102 g) - in Table 10.

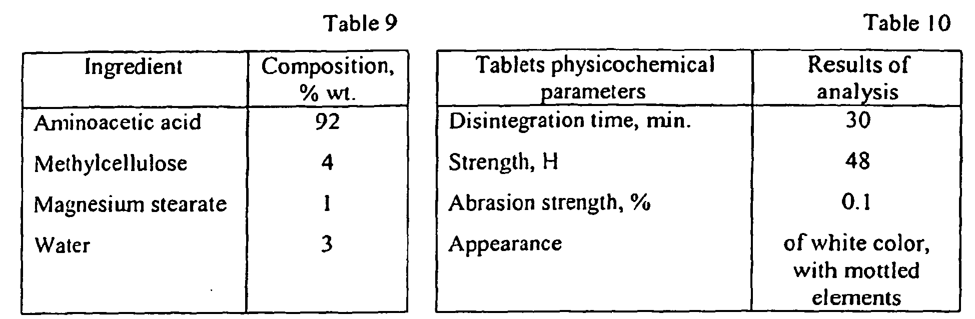

### Example 6

100 kg of aminoacetic acid are placed into fluidized bed apparatus for deposition of the film coating. The substance is moistened with 0.8% solution of methylcellulose (grade 100) for a period of 4-5 hours at bed temperature of 42 °C. After completion of the process, product is dried to a moisture content of 0.1 % wt., dusted with magnesium stearate in the amount of 0.1 % wt., after which fine fraction of aminoacetic acid is added as dispersant in the amount of 0.1 % wt.

Composition of the formulation obtained (tableting mixture) is presented in Table 11, and physicochemical properties of the tablets (6 mm, 0.102 g) - in Table 12.

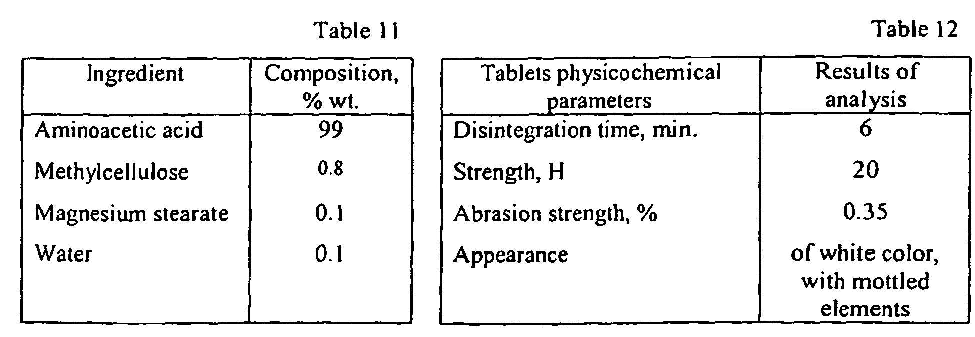

## Claims

1. Pharmaceutical formulation for preparation of prolonged release tablets comprising microcapsules containing non-agglomerated particles of water-soluble pharmaceutical drug coated with a layer of film-forming substance and lubricant and dispersant, wherein it additionally comprise water, content of said pharmaceutical drug and said film-forming substance in said microcapsules amounts to 96.0-99.2 % wt. and 0.8-4 % wt., correspondingly, and said pharmaceutical drug is used as dispersant, at following components ratio, % wt.:
| | |
|---|---|
| Microcapsules | 96.0 - 99.8 |
| Lubricant | 0.1 - 1 |
| Water | 0.1 - 3 |
with said dispersant being introduced in the amount of 0.1 to 10 % wt. of the mixture obtained.

2. Pharmaceutical formulation according to claim 1, wherein size of said non-agglomerated particles of said pharmaceutical drug amounts to 200-700 micron (µm).

3. Pharmaceutical formulation according to claims 1 or 2, wherein the portion of said non-agglomerated particles having size below 200 micron (µm) doesn't exceed 10 % wt.

4. Pharmaceutical formulation according to claims 1 or 2 or 3, wherein pharmaceutically acceptable substance capable of swelling is used as said film-forming substance.

5. Pharmaceutical formulation according to claims 1 or 2 or 3 or 4, wherein aminoacetic acid is used as said pharmaceutical drug.

6. Pharmaceutical formulation according to claims 1 or 2 or 3 or 4, wherein xylitol is used as said pharmaceutical drug.

7. Pharmaceutical formulation according to claims 1 or 2 or 3 or 4 or 5 or 6, wherein cellulose ethers soluble in water or in mixture of water with organic solvent are used as said film-forming substance.

8. Pharmaceutical formulation according to claims 1 or 2 or 3 or 4 or 5 or 6 or 7, wherein stearic acid or its pharmaceutically acceptable salt or their mixture is used as said lubricant.

9. Use of pharmaceutical formulation according to claims 1-8 as a mixture to compress tablets for sublingual application.

10. Method of preparation of pharmaceutical formulation according to claims 1-8 for obtaining a mixture for prolonged release tablets pressing, comprising preparation of microcapsules by deposition of coating of film-forming substance on non-agglomerated particles of water-soluble pharmaceutical drug, addition of lubricant and dispersant, **characterized by** that in the course of said microcapsules preparation and/or their intermediate storage moisture content in their coating is brought to concentration of not more than 0.5 % wt., said microcapsules are moistened with 0.1-3 % wt. of water to swell the coating prior to addition of said lubricant and said dispersant, and after introduction of said lubricant, said dispersant is added to the mixture in the form of fine fraction of said pharmaceutical drug.

11. Method according to claim 10, wherein non-agglomerated particles of said pharmaceutical drug are used having size of 200 to 700 micron (µm).

12. Method according to claim 10 or 11, wherein quantity of said non-agglomerated particles of said pharmaceutical drug having size below 200 micron (µm) used for deposition of the coating doesn't exceed 10 % wt.

13. Method according to claims 10 or 11 or 12, wherein deposition of polymeric coating on said non-agglomerated particles of said pharmaceutical drug is accomplished in fluidized bed apparatus.

14. Method according to claims 10 or 11 or 12 or 13, wherein pharmaceutically acceptable substance capable of swelling is used as said film-forming substance.

15. Method according to claims 10 or 11 or 12 or 13 or 14, wherein cellulose ethers soluble in water or in mixture of water with organic solvent are used as said film-forming substance.

16. Method according to claims 10 or 11 or 12 or 13 or 14 or 15, wherein stearic acid or its pharmaceutically acceptable salt or their mixture is used as said lubricant.

17. Method according to claims 10 or 11 or 12 or 13 or 14 or 15 or 16, wherein aminoacetic acid is used as said pharmaceutical drug.

18. Method according to claims 10 or 11 or 12 or 13 or 14 or 15 or 16, wherein xylitol is used as said pharmaceutical drug.

## Patentansprüche

1. Pharmazeutische Formulierung zur Herstellung von Tabletten mit verlängerter Freisetzung, umfassend Mikrokapseln, die nicht agglomerierte Teilchen eines wasserlöslichen Arzneistoffs enthalten, die mit einer Schicht aus einer filmbildenden Substanz überzogen sind, und ein Schmiermittel und ein Dispergiermittel, wobei sie zusätzlich Wasser umfasst, der Gehalt des Arzneistoffs und der filmbildenden Substanz in den MiKrokapseln in Entsprechung 96,0 - 99,2 Gew.-% und 0,8 - 4 Gew.-% beträgt, und der Arzneistoff als Dispergiermittel verwendet wird, bei folgendem Komponentenverhältnis, in Gew.-%:
| | |
|---|---|
| Mikrokapseln | 96,0-99,8 |
| Schmiermittel | 0,1 - 1 |
| Wasser | 0,1 - 3 |
wobei das Dispergiermittel in der Menge von 0,1 bis 10 Gew.-% der erhaltenen Mischung eingebracht wird.

2. Pharmazeutische Formulierung nach Anspruch 1, wobei sich eine Größe der nicht agglomerierten Teilchen des Arzneistoffs auf 200 - 700 Mikron (µm) beläuft.

3. Pharmazeutische Formulierung nach Anspruch 1 oder 2, wobei der Anteil der nicht agglomerierten Teilchen, die eine Größe unter 200 MiKron (µm) aufweisen, 10 Gew.-% nicht überschreitet.

4. Pharmazeutische Formulierung nach Anspruch 1 oder 2 oder 3, wobei eine pharmazeutisch unbedenkliche Substanz, die zum Anschwellen in der Lage ist, als die filmbildende Substanz verwendet wird.

5. Pharmazeutische Formulierung nach Anspruch 1 oder 2 oder 3 oder 4, wobei Aminoessigsäure als der Arzneistoff verwendet wird.

6. Pharmazeutische Formulierung nach Anspruch 1 oder 2 oder 3 oder 4, wobei Xylitol als der Arzneistoff verwendet wird.

7. Pharmazeutische Formulierung nach Anspruch 1 oder 2 oder 3 oder 4 oder 5 oder 6, wobei Celluloseether, löslich in Wasser oder in einer Mischung aus Wasser mit organischem Losungsmittel, als die filmbildende Substanz verwendet werden.

8. Pharmazeutische Formulierung nach Anspruch 1 oder 2 oder 3 oder 4 oder 5 oder 6 oder 7, wobei Stearinsäure oder ihr pharmazeutisch unbedenkliches Salz oder deren Mischung als das Schmiermittel verwendet wird.

9. Verwendung einer pharmazeutischen Formulierung nach den Ansprüchen 1 - 8 als Mischung, um Tabletten für sublinguale Anwendung zu pressen.

10. Verfahren zur Herstellung einer pharmazeutischen Formulierung nach den Ansprüchen 1 - 8 zwecks Erhalts einer Mischung für das Pressen von Tabletten mit verlängerter Freisetzung, umfassend die Herstellung von Mikrokapseln durch Aufbringung eines überzugs aus einer filmbildenden Substanz auf nicht agglomerierte Teilchen eines wasserlöslichen Arzneistoffs, die Zugabe eines Schmiermittels und eines Dispergiermittels, **dadurch gekennzeichnet, dass** im Verlauf der Herstellung der Mikrokapseln und / oder ihrer Zwischenlagerung der Feuchtigkeitsgehalt in ihrem Überzug auf eine Konzentration von nicht mehr als 0,5 Gew.-% gebracht wird, wobei die Mikrokapseln befeuchtet werden mit 0,1 - 3 Gew.-% Wasser zum Anschwellen des Überzugs vor Zugabe des Schmiermittels und des Dispergiermittels, und nach Einbringung des Schmiermittels das Dispergiermittel, in Form einer Feinfraktion des Arzneistoffs, der Mischung zugegeben wird.

11. Verfahren nach Anspruch 10, wobei nicht agglomerierte Teilchen des Arzneistoffs verwendet werden, die eine Größe von 200 bis 700 Mikron (µm) aufweisen.

12. Verfahren nach Anspruch 10 oder 11, wobei eine Menge der nicht agglomerierten Teilchen des Arzneistoffs mit einer Größe unter 200 Mikron (µm), die zur Aufbringung des Überzugs verwendet werden, 10 Gew.-% nicht überschreitet.

13. Verfahren nach Anspruch 10 oder 11 oder 12, wobei die Aufbringung eines polymeren Überzugs auf die nicht agglomerierten Teilchen des Arzneistoffs in einer Fließbettvorrichtung erreicht wird.

14. Verfahren nach Anspruch 10 oder 11 oder 12 oder 13, wobei eine pharmazeutisch unbedenkliche Substanz, die zum Anschwellen in der Lage ist, als die filmbildende Substanz verwendet wird.

15. Verfahren nach Anspruch 10 oder 11 oder 12 oder 13 oder 14, wobei Zelluloseether, löslich in Wasser oder in einer Mischung aus Wasser mit organischem Losungsmittel, als die filmbildende Substanz verwendet werden.

16. Verfahren nach Anspruch 10 oder 11 oder 12 oder 13 oder 14 oder 15, wobei Stearinsäure oder ihr pharmazeutisch unbedenkliches Salz oder deren Mischung als das Schmiermittel verwendet wird.

17. Verfahren nach Anspruch 10 oder 11 oder 12 oder 13 oder 14 oder 15 oder 16, wobei Aminoessigsaure als der Arzneistoff verwendet wird.

18. Verfahren nach Anspruch 10 oder 11 oder 12 oder 13 oder 14 oder 15 oder 16, wobei Xylitol als der Arzneistoff verwendet wird.

## Revendications

1. Formulation pharmaceutique pour la préparation de comprimés à libération prolongée comprenant des microcapsules contenant des particules non agglomérées de médicament pharmaceutique hydrosoluble enrobées d'une couche de substance filmogène, comprenant également un lubrifiant et un dispersant, la formulation comprenant en outre de l'eau, la proportion dudit médicament pharmaceutique et de ladite substance filmogène dans lesdites microcapsules s'élevant à 96,0 - 99,2 % en poids et a 0,8 - 4 % en poids, en correspondance, et ledit médicament pharmaceutique étant utilisé comme dispersant, avec les proportions de composants suivantes, exprimées en pourcentage en poids :
| | |
|---|---|
| Microcapsules | 96,0 - 99,8 |
| Lubrifiant | 0.1 - 1 |
| Eau | 0,1 - 3 |
ledit dispersant étant introduit dans une proportion allant de 0,1 à 10 % en poids relativement au mélange obtenu.

2. Formulation pharmaceutique selon la revendication 1, dans laquelle la taille desdites particules non agglomérées dudit médicament pharmaceutique va de 200 à 700 microns (µm).

3. Formulation pharmaceutique selon les revendications 1 ou 2, dans laquelle la proportion desdites particules non agglomérées dont la taille est inférieure à 200 microns (µm) ne dépasse pas 10 % en poids

4. Formulation pharmaceutique selon les revendications 1 ou 2 ou 3, dans laquelle on utilise à titre de ladite substance filmogène une substance pharmaceutiquement acceptable capable de gonfler.

5. Formulation pharmaceutique selon les revendications 1 ou 2 ou 3 ou 4, dans laquelle on utilise à titre dudit médicament pharmaceutique l'acide aminoacétique.

6. Formulation pharmaceutique selon les revendications 1 ou 2 ou 3 ou 4, dans laquelle on utilise à titre dudit médicament pharmaceutique le xylitol.

7. Formulation pharmaceutique selon les revendications 1 ou 2 ou 3 ou 4 ou 5 ou 6, dans laquelle on utilise à titre de ladite substance filmogène des éthers cellulosiques solubles dans l'eau ou dans un mélange d'eau et de solvant organique.

8. Formulation pharmaceutique selon les revendications 1 ou 2 ou 3 ou 4 ou 5 ou 6 ou 7, dans laquelle on utilise à titre dudit lubrifiant de l'acide stéarique ou son sel pharmaceutiquement acceptable ou leur mélange.

9. Utilisation de la formulation pharmaceutique selon les revendications 1 à 8 comme mélange pour produire des comprimés à application sublinguale.

10. Procédé de préparation d'une formulation pharmaceutique selon les revendications 1 à 8 permettant d'obtenir un mélange destiné à produire des comprimés à libération prolongée, comprenant la préparation de microcapsules mettant en oeuvre le dépôt d'un enrobage de substance filmogène sur des particules non agglomérées de médicament pharmaceutique hydrosoluble, l'ajout de lubrifiant et de dispersant, **caractérisé par le fait que**, au cours de la préparation desdites microcapsules et/ou de leur stockage intermédiaire on concentre la teneur en humidité de leur enrobage pour l'abaisser à 0,5 % en poids maximum, on hydrate lesdites microcapsules par 0,1 à 3 % en poids d'eau de manière à faire gonfler l'enrobage préalablement à l'ajout dudit lubrifiant et dudit dispersant, et suite à l'introduction dudit lubrifiant, on ajoute ledit dispersant au mélange sous la forme d'une fine fraction dudit médicament pharmaceutique.

11. Procédé selon la revendication 10, dans laquelle on utilise des particules non agglomérées dudit médicament pharmaceutique dont la taille va de 200 à 700 microns (µm).

12. Procédé selon la revendication 10 ou 11, dans laquelle la quantité desdites particules non agglomérées dudit médicament pharmaceutique dont la taille est inférieure à 200 microns (µm), utilisées pour le dépôt de l'enrobage, ne dépasse pas 10 % en poids.

13. Procédé selon les revendications 10 ou 11 ou 12, dans laquelle le dépôt d'un enrobage polymère sur lesdites particules non agglomérées dudit médicament pharmaceutique est accompli dans un appareil à lit fluidisé.

14. Procédé selon les revendications 10 ou 11 ou 12 ou 13, dans laquelle on utilise à titre de ladite substance filmogène une substance pharmaceutiquement acceptable capable de gonfler.

15. Procédé selon les revendications 10 ou 11 ou 12 ou 13 ou 14, dans laquelle on utilise à titre de ladite substance filmogène des éthers cellulosiques solubles dans l'eau ou un mélange d'eau et de solvant organique

16. Procédé selon les revendications 10 ou 11 ou 12 ou 13 ou 14 ou 15, dans laquelle on utilise à titre dudit lubrifiant de l'acide stéarique ou son sel pharmaceutiquement acceptable ou leur mélange.

17. Procédé selon les revendications 10 ou 11 ou 12 ou 13 ou 14 ou 15 ou 16, dans laquelle on utilise à titre dudit médicament pharmaceutique l'acide aminoacétique.

18. Procédé selon les revendications 10 au 11 ou 12 ou 13 au 14 ou 15 ou 16, dans laquelle on utilise à titre dudit médicament pharmaceutique le xylitol.
